# EUROPEAN PATENT APPLICATION

(11) **EP 1 251 181 A1**
(43) Date of publication of application: **23.10.2002**
(21) Application number: 01401026.8
(22) Date of filing: 20.04.2001
(51) Int. Cl.: C12Q 1/44, C12N 5/06, C12N 9/16

(54) **Non-recombinant cell lines capable of expressing predominantly cyclic nucleotide phosphodiesterase 4 (PDE4) subtype B and their use for the screening of PDE4 subtype-specific modulators**

(71) Applicant: WARNER-LAMBERT COMPANY, Morris Plains New Jersey 07950 (US)
(72) Inventor: Szilagyi, Corinne, 94400 Vitry-sur-Seine (FR); Jacob, Claire, 75014 Paris (FR)
(74) Representative: Dufresne, Guillaume Alain François

(57) **Abstract**

The invention concerns a non-recombinant cell line expressing predominantly a PDE4 subtype, more preferably PDE4B.

The invention also concerns a non-recombinant cell line capable, under appropriate conditions, of exhibiting an upregulated expression of a single target protein, preferably a PDE4 subtype, more preferably PDE4B.

The invention also concerns methods for the screening of a candidate molecule that modulates the expression or the activity of human phophodiesterase 4B.
The candidate molecules selected by the screening methods of the invention are potentially useful as therapeutic molecules for diseases caused or regulated by cyclic nucleotide-modulated transduction mechanisms, such as airway disorders like asthma.

## Description

### FIELD OF THE INVENTION

The invention relates to a non-recombinant cell line capable, under appropriate conditions, of selectively expressing majoritary a target protein, preferably an isoenzyme of PDE4B.

The invention also concerns methods for the screening of a candidate molecule that modulates the expression or the activity of human phosphodiesterase 4B.

### BACKGROUND OF THE INVENTION

Cyclic nucleotides are important second messengers that regulate and mediate a number of cellular responses to extra-cellular signals, such as hormones, light and neuro-transmitters.

The cyclic nucleotide phosphodiesterases regulate the cellular concentrations of cyclic nucleotides and thereby play a role in signal transduction. To date, eleven different classes of phosphodiesterases have been identified and, among them, phosphodiesterase 4 and 7 have been recognized as cAMP-specific enzymes.

Investigations of recent years revealed that isoenzymes of cyclic-3'-5'-nucleotide phosphodiesterases are critically important components of the cyclic-3',5'-adenosine monophosphate (cAMP) protein kinase A (PKA) signalling pathway.

The experimental data suggest that PDE isoenzymes are important in airway inflammation and that PDE inhibitors exert anti-inflammatory effects by acting on airway epithelial cells.

The type 4 phosphodiesterases are proteins encoded by 4 genes in mammals (PDE 4A, B, C, D) that encode multiple PDE isoforms generated from alternative spliced mRNA transcripts. However, the physiological significance of this diversity of cAMP specific PDE isoforms is not yet known, although it has been shown that the rat PDE4 genes have different patterns of expression in tissues (Houslay et al. ; 1998).

PDE4 isoenzymes are likely to be good targets for molecules acting in diseases caused or regulated by cyclic nucleotide-modulated transduction mechanisms. Pharmaceutical interest in this area has been further sparked by the hypothesis that different PDE isoenzymes having distinct sequences at regulatory and catalytic sites could allow the development of selective therapeutic agents that can target a specific cyclic nucleotide pool in a very limited number of cell types. This in turn holds great promise of being able to limit the toxic effects of many of the first generation selective PDE4 inhibitors.

Regulation of PDE4 gene expression appears to be a complex process. In various cell-based differentiation systems it has been demonstrated that elevation of intracellular cAMP levels plays a pivotal promoting role.

In general, PDE4 activity can be short-term or long-term regulated; short-term regulation of PDE4 activity is a cAMP-dependent protein kinase phosphorylation of the isoenzyme triggered by intracellular cAMP-elevating agents or a direct activation of PDE4 by phosphatidic acid . Long-term upregulation is also realized by increased intracellular cAMP concentrations but requires both protein and mRNA synthesis . An interesting feature is that different types of regulation can occur in the same cell-type. For example, cells possess a phospholipid-sensitive form of PDE4 which can be activated by anionic phospholipids (Houslay et al. ; 1998).

It has been shown that cAMP-increasing agents increased PDE4 activity in this cell-type by enhancement of the transcription of certain PDE4 subtypes (PDE4A and B) (Houslay et al. ; 1998).

There is a great need for new molecules that selectively modulate selectively the expression or the activity of the human PDE4 subtypes, and particularly molecules that are able to inhibit the expression or the activity of the human PDE4B.

### SUMMARY OF THE INVENTION

The invention concerns a non-recombinant cell line expressing predominantly the PDE4 B subtype with respect to the other PDE4 subtypes. In addition, this non-recombinant cell line exhibits, after an appropriate treatment, an upregulated expression of the PDE4 B subtype with respect to its level of expression before treatment.
Moreover the PDE4B protein represents in this non-recombinant cell line at least 80% of the total of PDE4 subtypes.
This non-recombinant cell line expresses a quantity of PDE4B enzyme which is at least 80 % of the total PDE4 quantity. Preferably, it expresses a quantity of PDE4B enzyme which is at least 90 % and most preferred between 95% and 99% of the total PDE4 quantity .
According to the present invention, the non-recombinant cell line has the neutrophile-like phenotype which corresponds to a NADPH-oxydase activity.
The non-recombinant cell line according to the invention is obtainable by growing an initial cell line with methyl sulfoxide and preferably with dimethylsulfoxide (DMSO) in the culture medium.
For the invention, the quantity of methyl sulfoxide or DMSO present in the culture medium is between 0.5 and 2.5 %, preferably this quantity is 1.3 % for a period of time ranging from 3 to 14 days and preferably 10 days.
In the non-recombinant cell line according to the present invention the cells are eucaryotic cells. Preferably, the cell line is a human cell line.
In the context of the present invention, the initial cell line to be used is a promyelocyte cell line and preferably the HL60 cell line.

The present invention also relates to the use of a cell line as described above for the screening of candidate molecules that modulate the expression or the activity of the PDE4B enzyme. Preferably, the said cell line is used for the screening of candidate molecules that inhibit the expression or the activity of the PDE4B enzyme.
In hence, the present invention also regards a method for the screening of a candidate molecule that modulates, preferentially inhibits, the expression or the activity of the PDE 4B enzyme, preferably the human PDE4B enzyme, said method comprising the steps of:
a) adding a candidate molecule to the culture medium of a population of cells according to anyone of claims 1 to 18;
b) harvesting the cells; and
c) quantifying the cAMP content in the cell lysate.
In this method the cAMP content measured in step (c) is compared with the cAMP content in the cell lysate of cells of the invention in the absence of the candidate molecule. Preferably, the quantification of cAMP content is performed on the cytosolic fraction of the cell lysed.

This screening method of the invention exploits the inventor's findings that a human promyelocytic cell line presents significantly upregulated expression of a specific subtype of phosphodiesterase, namely phosphodiesterase 4B, and a substantially downregulated expression of other PDE4 subtypes, preferably unmeasurable levels of all other PDE4 subtypes, when treated with a differentiating agent such as dimethylsulfoxide (DMSO).
A first screening method of the invention comprises the step of quantifying cAMP content in methyl sulfoxide-treated non-recombinant cells incubated with a candidate molecule to be assayed.
A second screening method of the invention comprises a step wherein an expression analysis of human phosphodiesterase 4B is performed. The candidate molecules selected by the screening methods of the invention are potentially useful as therapeutic molecules for diseases caused or regulated by cyclic nucleotide-modulated transduction mechanisms, airway disorders such as asthma, pulmonary hypertension, COPD (chronic obstructive pulmonary disease) and other inflammatory disorders including atopic dermatitis, psoriasis, Crohn's disease, ulcerative colitis and acute respiratory distress syndrome.
The invention also concerns a method for determining the selectivity of a candidate molecule for the PDE4B subtype, said method comprising:
a) adding a candidate molecule to the culture medium of a population of cells as described above and thus presenting a significantly upregulated expression of PDE4B and a substantially downregulated expression of other PDE4 subtypes, preferably unmeasurable levels of all other PDE4 subtypes;
b) quantifying the variation of cAMP contents in said culture
c) comparing variation of cAMP contents with other non-PDE4B selective candidate molecules .
Alternatively or in addition, the cAMP contents in step c) are compared to cAMP levels in the cell lysate of initial cells which have not been treated with methyl sulfoxyde or DMSO.
The invention also regards a kit for use in a method as describe above and comprising at least the cell line as described.

### BRIEF DESCRIPTION OF THE DRAWINGS

**Fig1:** Comparison of level of expression of PDE 4 subtypes in control cells and in DMSO-treated HL60 cells.
**Fig2:** Comparison by western blot of level of PDE4 subtypes proteins in control cells and in DMSO-treated HL60 cells.

### DETAILED DESCRIPTION OF THE INVENTION

The inventors have surprisingly found that a promyelocytic human cell line that normally expresses different phosphodiesterase 4 isoenzymes, such as PDE 4A, PDE 4B and PDE 4D, and mainly the PDE 4B isoenzyme, expresses exclusively the PDE 4B isoenzyme when treated with an appropriate concentration of a methyl sulfoxide, preferably dimethylsulfoxide.

This finding can be applied to test other non-recombinant cell lines to attempt to upregulate expression of selected proteins such as PDE4 isoenzymes.

In one of its preferred embodiments, the invention therefore comprises the method that was used to set up a particular PDE4B selective cellular assay. In this part of the invention it is described the characterization and quantification methods that were used to show differences in PDE4 subtype expression profiles in methyl sulfoxide-treated cells compared to control cells. This comprises the comparison by western blot of the phosphodiesterases 4 profiles in methyl sulfoxide-treated cells and in control cells. This also comprises the comparison of the enzymatic activity pattern of phosphodiesterase in methyl sulfoxide-treated cells and in control cells.

Thus, a particular embodiment of the invention concerns a method for the development of the PDE4B selective cellular assay for the screening of a candidate molecule that modulates the activity of the phosphodiesterase 4B, preferably the human PDE 4B. The method comprises the steps of:
a) incubating non-recombinant cells from a cell line such as the HL60 cell line in the presence of a methyl sulfoxide such as dimethyl sulfoxide (DMSO) without changing the culture medium during incubation,
b) harvesting the cultured cells and preferably rinsing many times the residual DMSO before further utilization,
c) counting the differentiated cells, preferably to use them either for the analysis of the PDE4 subtypes expression profile, or for determination of the PDE enzyme profiles.

Preferably, the methyl sulfoxide or the DMSO is present in the culture medium at a concentration ranging from 0.5 to 2.5 %, preferably from 1 to 1,5 % ; and most preferably, at a concentration of 1.3 %. The incubation time of the cells in the medium containing the methyl sulfoxide preferably ranges from 3 to 14 days, most preferably from 7 to 10 days. These concentrations and incubation times are not limited to the development of cell lines expressing a PDE4 subtype.

The PDE4 subtype transcripts found in DMSO-treated HL60 cells are preferably characterized, quantified and compared to control cells, through a method comprising the following steps:
a) incubating non-recombinant cells, preferably cells from a HL60 cell line, in the presence of a concentration of methyl sulfoxide, preferably dimethyl sulfoxide (DMSO), which is sufficient to significantly upgrade PDE4B expression in the cell;
b) harvesting the cultured cells and extracting the total RNA;
c) incubating the total RNA extract with random polynucleotide hexamers in the presence of an enzyme having a reverse transcriptase activity to generate cDNA;
d) amplifying the cDNA obtained in step c) with a pair of oligonucleotide primers that hybridize specifically to human PDE 4B cDNA;
f) detecting and quantifying the amplified nucleic acid.

In a preferred embodiment of the method described above, the detection and quantification of the PDE4 amplified nucleic acid comprises the steps of:
a) bringing into contact the amplified PDE4 nucleic acid with polynucleotide probes that hybridize specifically to the human PDE4 subtypes mRNA or to any nucleic acid strand of the human PDE4 subtypes cDNA;
b) detecting the nucleic acid hybrid complexes formed.

Preferably, the detection and quantification of amplified nucleic acid is performed with a polynucleotide probe, for example a probe that hybridizes specifically to human PDE 4B mRNA or to any nucleic acid strand of the human PDE 4B cDNA.

Some specific primers and probes that hybridize specifically with the PDE 4B mRNA or any strand of the human PDE 4B cDNA may be designed on the basis of the nucleic acid sequence of phosphodiesterase 4B that is disclosed in Bolger et al. (1993) incorporate herein by reference.

The primers and probes used for performing the characterization method described above can be prepared by any suitable method, including for example, cloning and restriction of appropriate sequences and direct chemical synthesis by a method such as the phosphodiester method of Narang et al. (1979), the phosphodiester method of Brown et al. (1979), the diethylphosphoramidic method of Boccage et al. (1981) and the solid support method described in EP 0 707 592. The disclosures of all these documents are herein incorporated by reference.

The PCR technology is the preferred amplification technique used in the present invention. The variety of PCR techniques are familiar to those skilled in the art.

For a review of PCR technology, see White (1997) and the publication entitled "PCR methods and applications" (1991, Cold Spring Harbor Laboratory Press).

PCR primers on either side of the PDE 4B nucleic acid sequences to be amplified are added to a suitably prepared nucleic acid sample along with dNTPs and a thermostable polymerase such as Taq polymerase.

The nucleic acid in the sample is denatured and the PCR primers are specifically hybridized to complement a nucleic acid sequence in the sample. The hybridized primers are extended. Thereafter, another cycle of denaturation, hybridization, and extension is initiated.

The cycles are repeated several times to produce an amplified fragment containing the nucleic acid sequence between the primer sites.

An illustrative embodiment of a PCR method that can be used for performing the characterization of the invention is described herein in the examples.

Preferably, the primers used to perform the amplification reaction of the characterization study of the invention are selected from the group consisting of the following nucleotide sequences : 5'-AGCTCATGACCCAGATAAGTG-3' and

5'-GCAGCGTGCAGGCTGTTGTGA-3' (Genebank L20966)

In this characterization study involving steps in which an expression analysis of the PDE 4B mRNA is performed, a quantity of PDE 4B mRNA or cDNA found in the control cells is compared to the quantity of PDE 4B mRNA or cDNA found in DMSO-treated HL60 cell cultures.

In a preferred embodiment of the characterization study described above, the detection and quantification of the PDE 4B amplified nucleic acid comprises the steps of:
a) bringing into contact the amplified PDE 4B nucleic acid with a polynucleotide probe that hybridizes specifically to the human PDE 4B mRNA or to any nucleics acids strand of the human PDE 4B cDNA;
b) detecting the nucleic acid hybrid complex formed.

Preferably, the detection probe is labelled.

In another embodiment of the characterization study described above, the amplified nucleic acid is labelled.

Any of the probes or primers of the present invention can be labelled if desired, by incorporating a label detectable by spectroscopic, photochemical, biochemical, immunochemical, or chemical means.

For example, useful labels include radioactive substances (³²P, ³⁵S, ³H, ¹²⁵I), fluorescent dyes (5-bromodesoxyuridin, fluorescein, acetylaminofluoren, digoxygenin) or biotin.

In a preferred embodiment, the detection probe used for performing the characterization study of the invention is labelled with biotin.

In another preferred embodiment of the characterization study of the invention, the amplified nucleic acid is labelled with digoxygenin.

A further preferred method of western blot to characterize the proteic profile of the human phosphodiesterases (PDE) 4 found in DMSO-treated HL60 cells compared to control cells, comprises the steps of:
a) Extracting cytosolic protein content from the DMSO-treated and control HL60 cells in an appropriate lysis buffer;
b) applying the extracted proteins onto an acrylamide electrophoresis gel to separate the different proteins;
c) transferring separated proteins from the acrylamide gel to a nitrocellulose membrane;
d) incubating the membrane with a blocking agent, then with the primary antibody (anti-PDE4 subtype-specific antibody), then with the secondary antibody (coupled to horse radish peroxidase for example);
e) revealing the PDE4 subtypes by incubating the membrane with a chemiluminescent substrate.

The cytosolic fraction of the cell lysate may be obtained by lysing the cells and particularly the DMSO-treated HL60 cells, for example by resuspending them in an appropriate lysis buffer, then by extracting the proteins and centrifuging the cell pellets in order to collect the supernatant which contains the cytosolic enzymes, optionally after filtration of the supernatant through a 0.2 µm filter.

Although it clearly appears from the examples of the present application that the expression of a main PDE4 isoenzyme and the downregulation of other PDE4 isoenzymes may not be achieved for all non-recombinant cell lines, the inventors findings provide appropriate guidelines for the skilled person to carry out suitable testing on a wide variety of potential candidates. The protein whose expression is to be regulated can vary substantially and is not intended to be restricted to PDE4 isoenzymes, although recombinant cell lines with the expression of a main PDE4 isoenzyme constitute preferred embodiments of the present invention.

A further preferred method to characterize the enzymatic activity of the human phosphodiesterases (PDE) found in DMSO-treated HL-60 cells compared to control cells, comprises the steps of:
a) Extracting cytosolic protein content from the DMSO -treated and control HL-60 cells in an appropriate lysis buffer;
b) applying the extracted proteins onto a FPLC column to separate the different phosphodiesterase activities;
c) collecting the separated FPLC fractions and preserving an aliquot from each one for PDE activity measurement;
d) measuring PDE activities extracted from DMSO -treated and control HL-60 cells using a modification of the method described by Smith et al. (1993);

In the methods described above, the human phosphodiesterase activities are measured both in cultures of DMSO -treated and in control HL-60 cells in order to compare the profile and the level of PDE activities in both cell lysates. The activity of human PDEs can be assessed by the capacity of the enzymes to hydrolyse several cyclic monophosphate nucleotides such as cAMP and cGMP; the level of cAMP hydrolysis is performed on the cytosolic fraction of the cell lysate.

The cytosolic fraction of the cell lysate may be obtained by lysing the cell and particularly the DMSO -treated HL60 cells harvested from the culture plates, for example by sonication, or by one or several cycles of freezing-thawing, then by extracting the proteins and centrifuging the cell pellets in order to collect the supernatant which contains the cytosolic enzymes, optionally after filtration of the supernatant through a 0.2 µm filter.

In another embodiment of this method, the quantification of the level of cAMP hydrolysis is performed on successive aliquot fractions from the chromatography eluate of the cytosolic fraction of the cell lysate.

Although it clearly appears from the examples of the present application that upregulation of specific protein expression may not be achieved for all non-recombinant cell lines, the inventors findings provide appropriate guidelines for the skilled person to carry out suitable testing on a wide variety of potential candidates. The protein whose expression is to be regulated can vary substantially and is not intended to be restricted to PDE4 isoenzymes, although recombinant cell lines with an upregulated PDE4 isoenzyme expression constitute preferred embodiments of the present invention.

In the preferred embodiment described above and in the examples, the inventors have found that cells from the human HL60 cell line mainly express the PDE 4B isoenzyme after treatement with dimethyl sulfoxide.

DMSO-treated non-recombinant cells such as HL60 cells represent valuable targets for screening candidate molecules that modulate the expression or the activity of PDE 4B and preferably the human PDE 4B. For instance, DMSO-treated HL60 cell cultures allow the skilled person to screen for molecules that regulate the synthesis of phosphodiesterase 4B and also of molecules that inhibit the activity of the PDE 4B isoenzyme.

The methods of the invention for the screening of candidate molecules represent an improvement as regards to prior art methods in that they use non-recombinant cell lines that are selective for the PDE 4B subtype. The term "selective for the PDE4B subtype" is intended to designate cell lines which produce significant or predominant levels of PDE4B subtype the production of other PDE4 subtypes are downregulated to the point that these enzymes become virtually unmeasurable in the cells. Significant levels of PDE4B subtypes means that in the cells of the cell line of invention the PDE4B protein represents at least 80% of the total of PDE4 subtypes.
Accordinlgy, the quantity of PDE4B enzyme expressed is non-recombinant cell line expressed is at least 80 % of the total PDE4 quantity. Preferably, it is expressed a quantity of at least 90 % and most preferred between 95% and 99% of the total PDE4 quantity .

Thus, a further object of the invention concerns a method for the screening of a candidate molecule that modulates the expression or the activity of the phosphodiesterase 4B and preferably the human PDE 4B. The method comprises the steps of:
a) Incubating non-recombinant cells, preferably cells from a HL60 cell line, in the presence of a concentration of a methyl sulfoxide, preferably dimethyl sulfoxide (DMSO), which is sufficient to significantly upregulate PDE4 B expression the said cells;
b) adding a candidate molecule to the culture medium in which the methyl sulfoxide-treated cells are cultured;
c) harvesting the cells; and
d) quantifying the cyclic AMP content in the cell lysate.
It is understood in the context of the present invention that the term modulate means inhibit, antagonize or block as well as stimulate, increase, facilitate the activity of PDE4B. A modulator can be a blocker or a stimulator.

In the screening method of the invention, the potency of a candidate molecule to inhibit the activity of human phosphodiesterase 4B is assessed by measuring cAMP content in cells stimulated by an adenylate cyclase activator.

In a preferred embodiment of the screening method described above, the inhibition of human phosphodiesterase 4B activity is measured in cultures of DMSO-treated HL60 cells in the presence or in the absence of the candidate molecule to be assayed. The cAMP content measured in both cell lysates are compared in order to determine the capacity of the candidate molecule to modulate, and more particularly inhibit, the activity of the human phosphodiesterase 4B.

More specifically, a method for the screening of a candidate molecule that modulate the activity of the phosphodiesterase 4B, preferably the human PDE 4B can comprise the following detailed process steps:
a) incubating cells such as cells from a HL60 cell line in the presence of dimethyl sulfoxide (DMSO);
b) adding a candidate molecule at different concentrations for a determined time to the culture medium wherein the DMSO-treated HL60 cells are cultured;
c) adding an adenylate cyclase activator to allow cAMP level to increase in the cells for a short time;
d) adding a stop solution to control the effect of the adenylate cyclase activator;
e) lysing the cells to collect their supernatant for cAMP measurement;
f) measuring cAMP content in each cell population treated with different concentrations of candidate molecule;
g) comparing the cAMP content in the DMSO-treated HL60 cells in the presence or absence of candidate molecule to test.

The inhibitory potency of the molecules is determined by the evaluation of the cAMP levels in the DMSO-treated HL60 cells when the molecule to test is added to the cell culture.

In this embodiment of the first screening method of the invention, the analysis of the cAMP contents is performed on the cytosolic fraction of the cell lysate.

The cytosolic fraction of a cell lysate can be obtained by lysing the cells, preferably DMSO-treated HL60 cells, for example by resuspending them in a lysis buffer, then by centrifuging the cell homogenate in order to discard the pellet (particulate fraction composed mainly by cell membranes and non-soluble entities) and to collect the supernatant. This supernatant contains the cytosolic and soluble components; preferentially the cyclic nucleotides.

More specifically, prior to lysis of the cells, the cells are centrifugated and washed with phosphate saline buffer (PBS). The cell lysate may be obtained by resuspending the cells in an appropriate lysis buffer; then the lysate can be collected for direct cAMP measurement or optionally can be frozen for further determination.

In the screening method described above, the level of cAMP in DMSO-treated HL60 cells incubated in the presence or in the absence of the candidate molecule is measured by an Enzyme Immuno-Assay (EIA), which is well known by one skilled in the art. The measurement of the protein content in each cells-containing well is also performed by spectrophotometric determination.

The present invention will be further illustrated, without being limited to, by the following examples.

### EXAMPLES:

### A) MATERIALS AND METHODS

### 1) MATERIALS

RPMI-1640 medium, fetal calf serum, L-glutamine, gentamycin, PBS were from Gibco/BRL (Life Technologies, Cergy Pontoise, France). DMSO, benzamidine hydrochloride, PMSF, SBTI, EDTA, EGTA, TRIS, bis-TRIS, DTT, bacitracine, BSA, TES, cAMP, acrylamide, ammonium sulfate, TEMED, SDS, ethidium bromide were from Sigma (St-Quentin-Fallavier, France). [³H]cAMP and [¹⁴C]AMP were from Amersham International. Rolipram was synthesized by the department of therapeutic chemistry in PGRD (Fresnes, France). Protease inhibitor cocktail tablets, first-strand cDNA synthesis kit, PCR-Dig labeling kit and PCR-ELISA Dig detection kit were from Roche-Boehringer-Mannheim (Meylan, France). Protein assay dye reagent was from Bio-Rad (Ivry-sur-Seine, France). ECL Plus Western Blotting Detection System was from Amersham Pharmacia Biotech (Orsay, France).

### 2) METHODS

### Reverse transcription and semi-quantitative RT-PCR

RNA was purified using Tripure™ Isolation Reagent, a single-step liquid phase separation issued from the total RNA isolation method developed by Chomczynski and Sacchi (1987). Washed and pelleted cells were resuspended at a concentration of 10⁶cells/ml Tripure and the total RNA extracted was quantified spectrophotometrically. RT-PCR was carried out using the commercial PCR ELISA Dig Labelling Kit. First strand cDNA was generated from 1µg of total RNA per sample using random hexamers to prime the reverse transcription (First Strand cDNA Synthesis Kit for RT-PCR) and was directly amplified by PCR following the addition of specific primer pairs.
Oligonucleotide primers were: PDE4A, 5'-TCGGAGCTGGCGCTCATGTAC-3' and 5'-GGCAGTGTGCTTGTCACACAT-3' (Genebank L20965) defining a 439-bp product; PDE4B, 5'-AGCTCATGACCCAGATAAGTG-3' and 5'-GCAGCGTGCAGGCTGTTGTGA-3' (Genebank L20966) defining a 327-bp product; PDE4C, 5'-CGCTCAGATAGCGACTATGAA-3' and 5'-TCCGCTTGAACTTGTTGGAGG-3' (Genebank L20968) defining a 331-bp product; PDE4D, 5'-GGCCTCCAACAAGTTTAAAA-3' and 5'-ACCAGACAACTCTGCTATTCTS-3' (Genebank L20970) defining a 339-bp product. The 5'-biotin-labelled capture probes used for the detection of PCR fragments were: 5'-AGCGCCAGCGCAGAGCCTAC-3' for PDE4A, 5'-TAGACCCCTAACATG-3' for PDE4B, 5'-AGCAGCCAAGCAGGGACCC-3' for PDE4C, 5'-CAATGTCTCAGATCAGTGGA-3' for PDE4D. Briefly, this PCR ELISA allows the specific detection of PCR products in a semi-quantitative microtiter plate format. The PCR products are labelled with digoxigenin during the amplification process (dig labelling). The labelled PCR products are analyzed by solution hybridization to a specific capture probe that is complementary to the inner part of the amplification product (dig detection). This capture probe is labelled with biotin to allow immobilization of the hybrid to a streptavidin coated microtiterplate surface. Unspecific amplification products will not hybridized to this capture probe and thus will be removed during the following washing steps; The bound hybrid is detected by an anti-digoxigenin peroxidase conjugate and by use of the colorimetric substrate ABTS. In comparison with suitable standards the color signal can be used for semi-quantitative evaluation of an amplification reaction. In our experiments the PDE4 subtypes PCR fragments amplified in the control cells (cells non treated with any of the compound tested), were our standards and the optical densities found for these PCR-fragments were normalized to 1.

### Cell Culture

The HL-60 cell line used in this study was obtained from the European Collection of Cell Culture (ECACC). HL60 cells were grown in RPMI 1640 medium supplemented with 20% (v/v) heat-inactivated calf serum at 37°C in an humidified atmosphere of 95% air-5% CO2. Culture media were changed every 3 to 4 days and the percentage of heat-inactivated calf serum was decreased to 10% (v/v) when DMSO (1.3%) was added. Since the addition of 1.3% DMSO, the culture medium was unchanged for 10 days. Cells in suspension were seeded at 10⁶ cells/ml; 10⁶ cells were used for mRNA extraction and 2.6x10⁸ cells for proteins extraction.

### Protein extraction and western blot analysis of PDE 4 subtypes

The cells were washed twice with sterile PBS and counted. After 5 min centrifugation at 2000 rpm the cell pellets (10⁷ cells) were frozen and stored at -85°C prior to use. Cell pellets were resuspended in a lysis buffer (10⁷ cells/0.1 ml): 10 mM Tris buffer, pH 7.4, containing 2 mM EGTA, 2 mM DTT, 1 mM PMSF, 1mM orthovanadate, 150mM NaCI, and one tablet/10ml of this lysis buffer of a protease inhibitor cocktail (0.3mg/ml antipain-HCL, 0.05mg/ml bestatin, 0.1mg/ml chymostatin, .3mg/ml E-64, 0.05mg/ml leupeptin, 0.05mg/ml pepstatin, 0.3mg/ml phosphoramidon, 2mg/ml pefabloc, 1mg/ml EDTA, 0.05mg/ml aprotinin). The homogenate was then centrifuged at 5000g for 20 min at 4°C. After centrifugation the pellet was discarded and the supernatant frozen at -80°C if not used immediately. The protein concentration of the supernatant was determined by the Coomassie Bradford method (1976) using Bio-Rad protein assay dye reagent.

An amount of 200µg proteins of the supernatant was submitted to a SDS-PAGE on a 10% SDS/14% polyacrylamide gel. After migration, proteins were transferred onto a nitrocellulose membrane (pore diameter = 0.45µM) for western blot analysis, as previously described (Towbin et al., 1992).

Unspecific binding sites were blocked with 5% nonfat dry milk in TBS, pH7.4 / 0.1% tween 20, for 1hour, at room temperature. The membrane was then incubated for 3hours at room temperature or overnight at 4°C with the primary antibody, directed against the PDE4 subtype of interest, diluted at the appropriate concentration in blocking buffer. The membrane was washed with TBS / 0.1% Tween 20 and incubated for 1 hour at room temperature with the HRP (Horseradish Peroxidase)-conjugated anti-rabbit secondary antibody at a 1:5000 in the blocking buffer. After last steps of washing, the membrane was incubated for 5min with ECL Plus Western Blotting Detection System to reveal the PDE4 subtypes.

### Protein extraction and chromatographic resolution of PDE activities

DMSO-treated cells along with the control cells were washed carefully at least twice with sterile PBS and counted. After 5 min centrifugation at 2000 rpm the cell pellets (2.5 x 10⁸ cells) were used immediately or frozen and stored at -85°C for later utilisation. Cell pellets were resuspended in an extraction buffer (10⁸ cells/4 ml): 20 mM bis-Tris buffer, pH 6.5, containing 2 mM EDTA, 2 mM DTT, 0.1 mM PMSF, 2 µg/ml SBTI, 100 µg:ml bacitracine and 2 mM benzamidine, and sonicated on ice. The homogenate was then centrifuged at 28000g for 60 min at 4°C. After centrifugation the pellet was discarded and the supernatant frozen at -80°C if not used immediately. The supernatant which contained the cytosolic enzymes was filtered through a 0.2 µm filter and applied onto a FPLC column (MonoQ HR 5/5, Pharmacia). The elution was performed at a flow rate of 1 ml/min with a linear gradient of NaCI (0-0.6 M) in a 20 mM bis-Tris buffer, pH 6.5, containing 2 mM EDTA, 2 mM DTT, 0.1 M PMSF and 2 mM benzamidine. Fractions of 1.5 ml were collected and stabilized by addition of BSA (1 mg/ml). An aliquot of each fraction was taken for further phosphodiesterase activities determination and the rest was stored at -85°C.

### Phosphodiesterase assay

Phosphodiesterase activities were assayed using a modification of the method described by Smith et al. (1993) utilizing the observation that 5'-nucleotides bind very strongly to alumina while the cyclic nucleotides are readily eluted at neutral or slightly alkaline pH. Assays were performed in 96-well plate at 37°C in a total volume of 200 µl. Each well contained 5 µl of each eluted fractions and assay buffer (final concentration of 40 mM Tris-HCI, pH8.0, 5 mM MgCl₂, 4 mM β-mercaptoethanol, 10 nM [³H]cAMP and 10 µM cAMP); for the identification of the phosphodiesterase activities found, cGMP was also used to replace cAMP as a substrate and Ca²⁺ (2 mM), calmodulin (4 units/well), cGMP (10 µM) and rolipram (30 µM) were used as modulators of activity. Following 60 min incubation, the reactions were terminated by the addition of [¹⁴C]AMP (1 µM) in TFA (0.5%). Membrane-bottomed microtiter plates (Silent monitor, Nalge Nunc) filled with 50 mg alumina were equilibrated with 0.1M TES-NaOH, pH 8.0. The reaction mixtures were applied to these "alumina micro-columns" and the non-hydrolyzed cAMP was eluted with 3 ml equilibration buffer. The [³H]AMP and [¹⁴C]AMP were then eluted with 2 ml NaOH directly into scintillation vials containing alkali-compatible scintillant (Ultima gold, Packard). The separated [³H]cAMP was back-corrected for recovery using the separated and non-separated ¹⁴C values and expressed as a fraction of the total [³H]cAMP to give the amount of substrate hydrolyzed.

### Cell culture for the screening of candidate molecules

HL-60 cells were grown in suspension in culture flasks, and were seeded at a concentration of 1.5x10⁶ cells/ml in fresh culture medium before addition of 1.3% DMSO. The flasks were incubated for 7 to 10 days at 37°C in an humidified atmosphere of 95% air-5% CO₂. After this differentiation period, the cell cultures were centrifugated to pellet the cells and remove the medium by aspiration. The cell pellets were washed at least 2 times with PBS. The cells were then suspended at a concentration of 1.5x10⁶ cells/ml in the incubation buffer constituted by 150mM Tris/HCl buffer, 2mM EDTA, pH 7.4 and aliquots of this suspension were distributed in dosage tubes. The candidate molecules were added in each tube diluted in 150mM Tris/HCl buffer, 2mM EDTA, pH 7.4 to reach the desired concentrations to test. The cells were then incubated for 30 min at 37°C under gentle shaking before addition of 10µM forskolin, adenylate cyclase activator, for 15 min. After the activation time, the tubes were stored on ice and 200 µl of stop solution, composed by 0.2M HCI, 1% Triton X100, 0.2 mM IBMX, was added in each tube. The cells were then disrupted by sonication to allow the measurement of intracellular cAMP produced during the reaction.

### cAMP measurement

cAMP levels in each cell population treated with candidate molecule were evaluated using an EIA kit from Euromedex. This kit is a competitive immunoassay for the quantitative determination of CAMP in samples treated with 0.1 M HCI. The kit uses a polyclonal antobody to cAMP to bind, in a competitive manner, the cAMP in the sample or an alkalin phosphatase molecule which has cAMP covalently attached to it. 280 µl of each sample are transferred to 96-wells plate to separate cellular cytosol from particulate fraction by centrifugation, 15 min at 3500 rpm, 4°C. The cytosolic fraction separated from the pellet is then transferred to a "transfer 96-wells plate", which is stored on ice until protein and cAMP measurement. 195 µl of each sample along with samples from the cAMP standard curve provided in the kit, are acetylated with 10 µl of a mix of 1:1/2 triethylamine : anhydrous acetic acid before competitive cAMP-EIA.

### EXAMPLE 1

### Comparison of the expression profile of PDE4 subtypes in DMSO-treated HL60 cells versus HL60 control cells

Cells were cultured and treated 10 days with 1.3% DMSO. The control cells were cultivated in the same time without DMSO. After treated, the cells were washed and prepared for mRNA extraction as described in the Materials and Methods section. Briefly, One µg of total RNA from control cells (HL60) and DMSO-treated HL-60 cells (HL60/DMSO) was reverse transcribed and amplified with subtype specific PDE4 primers.The analysis of the PCR fragments was performed by electrophoresis on 1.5% agarose gels and visualized by ethidium bromide staining. PCR fragments were quantified by semi-quantitative PCR.

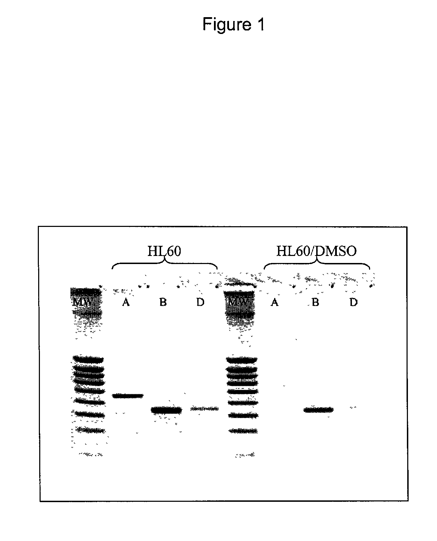

Changes in PDE4 subtype expression were evaluated by semi-quantitative PCR after the treatment of the cells with DMSO and compared to control cells. The results showed that PDE4C was not found in treated or untreated HL60 cells. The results obtained for the 3 other subtypes (A, B and D) are shown in figure 1. In untreated HL60 cells, transcripts corresponding to the 3 subtypes A, B and D were found, with a predominance of PDE4B. In DMSO-treated HL60 cells, PDE4B was the main expressed subtype, while PDE4A was not detected and PDE4D was weakly expressed.

### EXAMPLE 2

### Evaluation by western blot analysis of levels of PDE4 subtypes in HL60 cells and in DMSO-treated HL60 cells

After the 10-days treatment with DMSO the cells were washed and prepared for proteins extraction as described in the Material and Method section. Spots on the western blots corresponding to the PDE4 subtypes were quantified by densitometric analysis.

The analysis and identification of the PDE4 subtypes were performed using specific polyclonal antibodies : AC55 (1/500), K116 (1/2000) and K118 (1/2000) were used to detect PDE4A, PDE4B and PDE4D, respectively.

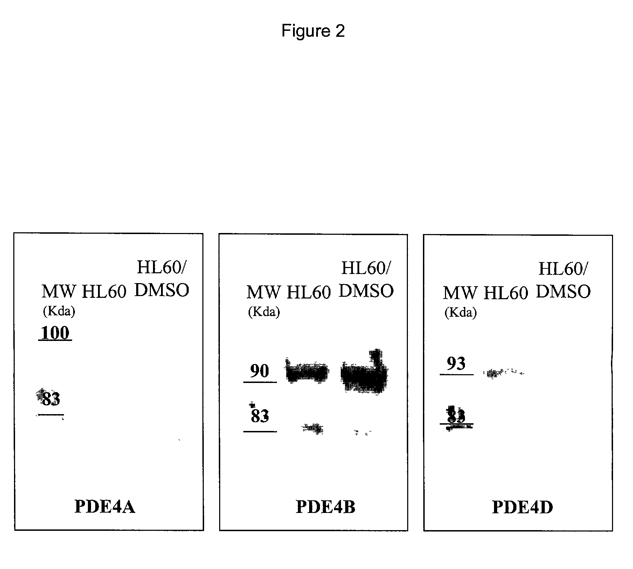

Changes in PDE4 subtypes expression were evaluated by densitometry after the treatment of the cells with DMSO and compared to the control cells. The results obtained for the each subtype (A, B and D) are shown in figure 2. PDE4A and PDE4D isozymes are downregulated in DMSO-treated HL60 cells when compared to control cells; moreover in DMSO-treated cells PDE4D is no more detectable while a very low expression corresponding to PDE4A is still present. I opposite, PDE4B is stongly upregulated after the treatment and is unequivocally majoritary. These data are well correlated with the data obtained at the mRNA level for the different subtypes.

### EXAMPLE 3

### Separation and identification of PDE activities found in control and treated cell lines

We showed by mRNA and proteins analysis that in HL-60 cells a treatment with 1.3% DMSO for 10 days was able to induce a significant decrease of PDE4A and 4D expression leading these cells to majoritary express PDE4B. A significant increase of the total PDE4 activity along with the disappearance of a cAMP-hydrolysing activity, probably due to PDE4A and D down regulation, was also seen in these cells.

### EXAMPLE 4

### Utilization of the DMSO-treated HL-60 cells to assess the inhibitory activity of a candidate molecule : measurement of cAMP increase in the cells incubated with Rolipram.

HL-60 cells were used in this experiment as a PDE4B selective PDE4 subtype assay, to assess the inhibitory potency of rolipram, a well-known PDE4 inhibitor. As can be seen in figure 4 we obtained a classical dose-response curve; cAMP content increased when we increased the concentrations of rolipram used for PDE4B inhibition. We reached a plateau corresponding to a maximum cAMP release.

### References:

- Beaucage et al., 1981, *Tetrahedron Lett,* **22**: 1859-1862
- Bolger et al., 1993, *Molec. Cell Biol.*, **13**: 6558-6571
- Bradford MM, 1976, *Anal Biochem,* **72**:248-54
- Brown EL, Belagaje R, Ryan MJ, Khorana HG, 1979, *Methods Enzymol.,* **68**:109-151
- Chomczynski, P. and Sacchi N., 1987, *Anal. Biochem.,* **162**: 156-159.
- Houslay et al., 1998, *Advances in Pharmacol.*, **44** :225-342.
- Livi, G. P.; Kmetz, P.; McHale, M. M.; Cieslinski, L. B.; Sathe, G. M.; Taylor, D. P.; Davis, R. L.; Torphy, T. J.; Balcarek, 1990, Molec. Cell. Biol. **10**: 2678-2686.
- Narang SA, Hsiung HM, Brousseau R, 1979, *Methods Enzymol.,* **68**:90-98
- White, M.B. et al., 1997, *Genomics,* **12**:301-306.
- Smith et al., 1993, *Anal. Biochem.,* **241**: 355-357.
- Towbin H, Staehelin T, Gordon J, 1992, *Biotechnology*, **24**:145-9.

## Claims

1. A non-recombinant cell line **characterized in that** it expresses predominantly the PDE4 B subtype with respect to the other PDE4 subtypes.

2. A non-recombinant cell line **characterized in that** it exhibits, after an appropriate treatment, an upregulated expression of the PDE4 B subtype with respect to its level of expression before treatment.

3. A non-recombinant cell line according to claims 1 or 2, **characterized in that** PDE4B protein represents at least 80% of the total PDE4 subtypes.

4. A non-recombinant cell line according to any one of claims 1 to 3, expressing a quantity of PDE4B enzyme which is at least 80 % of the total PDE4 quantity.

5. A non-recombinant cell line according to any one of claims 1 to 4, expressing a quantity of PDE4B enzyme which is at least 90 % of the total PDE4 quantity.

6. A non-recombinant cell line according to any one of claims 1 to 5, expressing a quantity of PDE4B enzyme which is between 95% and 99% of the total PDE4 quantity .

7. A non-recombinant cell line according to any one of claims 1 to 6, having the neutrophile-like phenotype.

8. A non-recombinant cell line according to claim 7, wherein the neutrophile-like phenotype corresponds to a NADPH-oxydase activity.

9. A non-recombinant cell line according to any one of the claims 1 to 8, obtainable by growing an initial cell line with methyl sulfoxide in the culture medium.

10. A non-recombinant cell line according to any one of claims 1 to 8, obtainable by growing an initial cell line with dimethylsulfoxide (DMSO) in the culture medium.

11. A non-recombinant cell line according to claim 9 or 10, wherein the methyl sulfoxide or the DMSO is in a quantity of between 0.5 and 2.5 %.

12. A non-recombinant cell line according to claim 11, wherein the methyl sulfoxide or the DMSO is in a quantity of 1.3 %.

13. A non-recombinant cell line according to any one of claims 9 to 12, wherein the methyl sulfoxide or the DMSO is present in the medium culture for a period of time ranging from 3 to 14 days.

14. A non-recombinant cell line according to claim 13, wherein the methyl sulfoxide or the DMSO is in the medium culture for 10 days.

15. A non-recombinant cell line according to anyone of claims 1 to 14, wherein the cells are eucaryotic cells.

16. A non-recombinant cell line according to anyone of claims 9 or 10, wherein the initial cell line is a promyelocyte cell line.

17. A non-recombinant cell line according to claim 16, wherein the initial cell line is HL60.

18. A non-recombinant cell line according to any of the claims 1 to 17, which is a human cell line.

19. Use of a cell line according to anyone of claims 1 to 18 for the screening of candidate molecules that modulate the expression or the activity of the PDE4B enzyme.

20. Use according to claim 19 for the screening of candidate molecules that inhibit the expression or the activity of the PDE4B enzyme.

21. A method for the screening of a candidate molecule that modulates, preferentially inhibits, the expression or the activity of the phosphodiesterase 4B (PDE 4B), said method comprising the steps of:
a) adding a candidate molecule to the culture medium of a population of cells according to anyone of claims 1 to 18;
b) harvesting the cells; and
c) quantifying the cAMP content in the cell lysate.

22. The method of claim 21, wherein the cAMP content measured in step (c) is compared with the cAMP content in the cell lysate of cells according to anyone of claims 1 to 18 in the absence of the candidate molecule.

23. The method of claim 21 or 22, wherein the quantification of cAMP content is performed on the cytosolic fraction of the cell lysed.

24. The method of screening according to anyone of the claims 21 to 23, wherein the candidate molecule modulates, preferentially inhibits, the expression or the activity of the human phosphodiesterase 4B.

25. A method for determining the selectivity of a candidate molecule for the PDE4B subtype, said method comprising:
a) adding a candidate molecule to the culture medium of a population of cells according to anyone of claims 1 to 18;
b) quantifying the variation of cAMP contents in said culture
c) comparing variation of cAMP contents with other non-PDE4B selective candidate molecules .

26. A method according to claim 25, wherein in step c) the cAMP contents are compared to cAMP levels in the cell lysate of cells according to claim 16 or 17 which have not been treated with methyl sulfoxyde or DMSO.

27. A kit for use in a method according to anyone of the preceeding claims.
